# EUROPÄISCHE PATENTANMELDUNG

(11) **EP 1 522 599 A1**
(43) Veröffentlichungstag der Anmeldung: **13.04.2005**
(21) Anmeldenummer: 04104549.3
(22) Anmeldetag: 21.09.2004
(51) Int. Cl.: C12Q 1/68

(54) **Verwendung von an metaRING bindenden Substanzen zur Diagnose und Behandlung von Krebs**

(30) Priorität: 22.09.2003 DE 10345011
(71) Anmelder: Hinzmann, Bernd, Dr., 13127 Berlin (DE); Rosenthal, André, Prof., 14482 Potsdam (DE); Heiden, Esmeralda, 10589 Berlin (DE); Hermann, Klaus Dr., 12679 Berlin (DE)
(72) Erfinder: Hinzmann, Bernd, Dr., 13127 Berlin (DE); Rosenthal, André, Prof., 14482 Potsdam (DE); Heiden, Esmeralda, 10589 Berlin (DE); Hermann, Klaus Dr., 12679 Berlin (DE)

(57) **Zusammenfassung**

Die Erfindung betrifft das neue Protein Metaring und Verwendungen von Metaring zur Diagnose und Behandlung von Krebs, sowie zum Screenen nach Substanzen für solche Zwecke.

## Beschreibung

### Technisches Gebiet

### Gebiet der Erfindung

Die Erfindung betrifft ein neues RING Finger Protein, hiermit Metaring genannt, eine hierfür codierende Nukleinsäure, Verwendungen von Metaring oder daraus abgeleiteten Sequenzen zum Screenen nach daran bindenden Substanzen, sowie die Verwendung von an Metaring bindenden Substanzen zur Diagnose und/oder Behandlung von Krebs, insbesondere von Kolon- Rectum-, Lungen-, Magen-, Brust-, Ovar-, und/oder Uterustumoren.

### Stand der Technik

Hintergrund der Erfindung und Stand der Technik

RING Finger Proteine enthalten das Sequenzmotiv Cys-X2-Cys-X9-39-Cys-X1-3-His-X23-Cys/His-X2-Cys-X4-48-Cys-X2-Cys, wobei X jede beliebige Aminosäure sein kann. Neben den stark konservierten Cystein (Cys) und Histidin (His) Resten ist die weitere Sequenz der RING Finger Proteine nur wenig konserviert. Das RING Finger Sequenzmotiv ist an der Bindung von Zink-Ionen beteiligt.

Es gibt umfangreiche Hinweise darauf, dass RING Finger Proteine eine wichtige Rolle bei der Ubiquitylierung von Proteinen spielen. An der Ubiquitylierung sind drei verschiedene Enzyme beteiligt: Das E1 Ubiquitin-aktivierende Enzym, E2 Ubiquitin-konjugierende Enzyme und E3 Ubiquitin Protein Ligasen (Freemont PS, Current Biology (2000) 10, R84-R87; Joazeiro CAP and Weissman AM, Cell (2000) 102, 549552). Für viele RING Finger Proteine konnte eine E3 Ubiquitin Ligase Aktivität bzw. die Beteiligung an einem Multi-Protein E3 Ligase Komplex nachgewiesen werden. E3 Enzyme binden an ein E2 Enzym und werden für die Erkennung des Substrates und die Übertragung des Ubiquitins auf das Substrat benötigt. Eine Ubiquitinylierung mit einer Kette von mindestens vier Ubiquitinresten markiert ein Protein für die Degradation durch das 26S Proteasom, während eine Übertragung von einzelnen Ubiquitinresten die Aktivität von Proteinen verändern kann, so z.B. die subzelluläre Lokalisation oder die Interaktion mit anderen Proteinen. Sowohl die Degradation von Proteinen, als auch die Veränderung der Aktivität eines Proteins kann vielfältige Auswirkungen auf biologische Prozesse wie Zellproliferation, Apoptose, Zellzyklus, Transkription usw. haben (Freemont PS, Current Biology (2000) 10, R84-R87Joazeiro CAP and Weissman AM, Cell (2000) 102, 549-552; Jackson PK et al., Trends in Cell Biology (2000),10, 429-439).

Auch in der Tumorgenese spielen RING Finger Proteine eine wichtige Rolle. So führt der Verlust der RING Finger Funktion des Tumor Suppressor Gens BRCA1 zu einer Deregulation des Wachstums; und Mutationen in der RING Finger Domäne von BRCA1 findet man in familiär gebundenen Brust und Ovar Tumoren (Joazeiro CAP and Weissman AM, Cell (2000) 102, 549-552).

Krebs ist eine meist letal verlaufende Erkrankung mit zunehmender Inzidenz. Daher ist es wünschenwert, verbesserte Ansätze zur Diagnose und Therapie von Krebserkrankungen zur Verfügung zu stellen.

### Kurze Beschreibung der Zeichnungen

Fig. 1: Chipanalyse zur differentiellen Expression von Metaring in Colontumorgewebe aus 20 Patienten,

Fig. 2: Krebsgewebe Profiling (CPA)

Fig. 3: in situ Hybridisierung von Kolon Tumorgewebe

Fig. 4: in situ Hybridisierung von Kolon Tumorgewebe

Fig. 5: in situ Hybridisierung von Kolon Normalgewebe

### Ausführungsform(en) der Erfindung

Technisches Problem der Erfindung

Der Erfindung liegt daher das technische Problem zugrunde, pharmazeutische Zusammensetzungen zur Diagnose, auch zur Verlaufs- bzw. Progressionsprognose, und/oder zur Behandlung von Krebs, insbesondere der eingangs genannten Tumore, anzugeben sowie Mittel zu deren Identifizierung.

Grundzüge der Erfindung und bevorzugte Ausführungsformen.

Zur Lösung dieses technischen Problems lehrt die Verwendung einer für Metaring codierenden Nukleinsäure und/oder eines Metaring Peptids oder Proteins zur Detektion von Krebs oder zur Detektion eines Risikos der Erkrankung an Krebs oder zur Detektion eines Risikos einer Progression einer Krebserkrankung, wobei eine Gewebeprobe (des zu untersuchenden Gewebetypes bzw. Krebstypes, beispielsweise Ovartumor) auf Transkription oder Übertranskription von Metaring RNA oder auf Expression oder Überexpression eines Metaring Proteins untersucht wird. Eine an für Metaring codierende Nukleinsäure oder eine an Metaring Protein oder Peptid bindende Detektorsubstanz, vorzugsweise enthaltend eine Reportergruppe, kann verwendet werden, wobei Bindung besagter Nukleinsäure und/oder besagten Proteins oder Peptids an die Detektorsubstanz halbquantitativ oder quantitativ detektiert wird. In diesem Zusammenhang lehrt die Erfindung weiterhin ein Testsystem zur (in vitro) Detektion eines eingangs genannten Karzinoms oder eines Risikos der Erkrankung hieran oder der Progressionsprognose, enthaltend Mittel zur quantitativen Messung der Expression von Metaring in Gewebeproben, wobei diese Mittel beispielsweise Mittel zur Amplifikation und spezifischen Detektion von Metaring RNA und/oder eine Detektorsubstanz, insbesondere spezifisch für Metaring Protein, sein können.

Die Erfindung lehrt weiterhin die Verwendung einer Metaring RNA oder eines Metaring Proteins oder Peptids zum Screenen nach daran bindenden Substanzen, insbesondere prospektiven Wirkstoffen zur Modulierung, insbesondere Inhibierung, von besagter RNA oder besagtem Protein oder Peptid, oder prospektiven Detektorsubstanzen, wobei eine prospektive Substanz oder eine Mischung solcher prospektiver Substanzen mit besagter RNA oder besagtem Protein oder Peptid kontaktiert wird, wobei mit einem Bindungsassay Bindungsereignisse festgestellt werden, und wobei eine bindende prospektive Substanz, ggf. nach Dekonvolutierung, selektiert wird. In diesen Zusammenhängen lehrt die Erfindung weiterhin ein Screeningsystem zur Ermittlung von für die Behandlung von vorstehenden Tumorerkrankungen geeigneten Wirksubstanzen enthaltend eine Metaring Nukleinsäure oder ein Metaring Protein bzw. Peptid, Mittel zur Bestimmung von (in vitro) Bindungsereignissen an die Metaring Nukleinsäure oder an das Metaring Protein bzw. Peptid, und/oder Mittel zur Bestimmung der (in vitro) Aktivität von Metaring Protein. Hierbei kann Metaring in einem zellfreien oder einem zellbasierten System, letzteres insbesondere aufweisend Zellen aus vorstehend genannten Geweben bzw. eine hieraus entwickelte Zelllinie, vorliegen. Mittel zur Bestimmung von Bindungsereignissen können beispielsweise natürlicherweise in normalen oder in Tumorzellen z.B. an Metaring Protein bindende Substanzen bzw. Assoziationspartner umfassen, wobei über deren (freie) Konzentration bzw. Konzentrationsänderung bei Zugabe prospektiver Wirksubstanzen und/oder Detektorsubstanzen eine kompetitive Bindung einer bindenden Wirk- oder Detektorsubstanz bestimmt wird. Solche Mittel können aber auch physikalische bzw. physikalisch-chemische Methoden umfassen, wie beispielsweise Röntgenstrukturanalyse und/oder NMR, insbesondere zweidimensionale 1 H/1 H oder 15N/1H oder 14C/1H Korrelationsspektroskopie. Hierbei werden Spektren vor und nach der Zugabe einer prospektiven Wirk- oder Detektorsubstanz miteinander verglichen und im Falle von Änderungen ist ein Bindungsereignis festgestellt. Es kann mit Spektren oder dergleichen entweder von Metaring oder der prospektiven Substanz oder mit einer Kombination aus beidem gearbeitet werden. Selbstverständlich sind auch alle anderen fachüblichen Methoden der Bestimmung von Bindungsereignissen und/oder Proteinaktivitäten einsetzbar. Beispielsweise kann eine prospektive Substanz (oder mehrere Substanzen, räumlich voneinander getrennt) immobilisiert sein, wobei dann markiertes Metaring aufgetragen wird. Ein Bindungsereignis wird dann nach Auftrag und folgender Spülung durch Detektion, ggf. ortlich aufgelöst, der Markierung gebundenen FABP4s festgestellt. Bindungsereignisse können auch ohne Markierung eines der Bindungspartner mittels der dem Fachmann geläufigen Biacore Technologie (Oberflächenplasmonen Resonanz)detektiert werden. Umgekehrt kann Metaring immobilisiert sein und es wird eine markierte prospektive Substanz oder eine Mischung hieraus aufgetragen. Bindungsereignisse werden analog der vorstehenden Variante festgestellt.

Die Erfindung lehrt schließlich die Verwendung einer Metaring inhibierenden oder daran bindenden Substanz zur Herstellung einer pharmazeutischen Zusammensetzung zur Behandlung und/oder Diagnose von Krebs, insbesondere eines der eingangs genannten Tumore.

Die Substanz kann ein Antikörper sein, welcher durch Immunisierung eines nicht-menschlichen Säugetiers mit einem Metaring Peptid oder Protein, mit hierfür codierender cDNA transfizierte Zellen, cDNA Immunisierung (Genovac), mit endogen ein solches Peptid oder Protein exprimierenden Tumorzellen, oder mit rekombinant hergestellten Metaring Peptiden oder Proteinen, erhältlich ist, oder ein Phage-Display-Antikörper sein. Die Substanz kann aber auch eine Mimikryverbindung eines Antikörpers gegen ein Metaring Peptid oder Protein sein. Die Substanz kann schließlich ein Aptamer, eine antisense RNA, ein Ribozym oder eine siRNA gegen Metaring Nukleinsäuren sein. Die Substanz kann zusätzlich eine zytotoxische und/oder immunstimulierende Komponente tragen.

Bevorzugt ist es, wenn die vorstehenden, an Metaring Protein bindenden Substanzen in der Verwendung zu therapeutischen Zwecken spezifisch an das Metaring Protein binden und es in seiner biologischen Aktivität modulieren. Dies ist nicht erforderlich im Falle der Fusion bzw. Verbindung der Substanz mit einer zytotoxischen Komponente. Dies ist weiterhin nicht erforderlich, wenn die Substanz der Gewinnung eines anti-idiotypischen Antikörpers dient, welcher vom Immunsystem eines Patienten aufgrund seiner nicht-humanisierten Form als körperfremd erkannt wird und dem Immunsystem ansonsten ein Metaring-Antigen präsentiert.

Die pharmazeutische Zusammensetzung kann zur beliebigen Applikation, beispielsweise i.v. oder i.p. Injektion, hergerichtet sein. Eine Herrichtung zur lokalen Applikation in Tumorzellen enthaltendem Gewebe wird sich empfehlen im Falle des Einsatzes einer zytotoxischen Komponente.

Die Erfindung läßt sich im Rahmen eines Verfahrens zur Diagnose bzw. (Progressions-) Prognose einer Tumorerkrankung verwenden, wobei eine Detektorsubstanz in einer Ausführungsform mit einer Reportergruppe in zu untersuchendes Gewebe, ggf. in vitro nach Gewebeentnahme, appliziert wird, wobei das zu untersuchende Gewebe dann einer Detektionsverfahrenstufe unterworfen wird, welche sensitiv für die Reportergruppe ist, und wobei im Fall der Detektion eines definierten Mindestwertes der Reportergruppe im Gewebe das Gewebe als Tumorzellen enthaltend qualifiziert bzw. als progressionsgefährdet oder nicht progressionsgefährdet eingestuft wird, sowie eines Verfahrens zur Behandlung einer Krebserkrankung, wobei eine erfindungsgemäße pharmazeutische Zusammensetzung in einer physiologisch wirksamen Dosis einem Patienten dargereicht wird. Im Falle der Diagnose bzw. Progressionsprognose kann zusätzlich oder alternativ eine Gewebeprobe mit einem erfindungsgemäßen Testsystem auf Metaring Expression untersucht werden.

Die Erfindung beruht insbesondere auf der Erkenntnis, daß Metaring in diversen Geweben unterschiedlich exprimiert wird, i.e. in den Tumorgeweben ist die Expression im Falle solcher Karzinome höher, verglichen mit normalen Zellen gleichen Gewebes und der daraus herleitbaren technische Lehre, daß Metaring als Zielmolekül bei der Diagnostik und Therapie bzw. Prophylaxe eingangs genannter Tumorerkrankungen eingesetzt werden kann. Metaring kann also als spezifischer Marker zur Identifizierung von Tumorzellen in den besagten Tumorgeweben dienen. Es ist erkannt worden, dass pathologisch normale Zellen mit einer erhöhten Expression ein erhöhtes Risiko aufweisen, zu Krebszellen zu transformieren. Auf der anderen Seite bietet die Inhibierung von Metaring die Möglichkeit, in die Tumor-spezifischen Metaring Assoziationen mit anderen Prozessen in den Tumorzellen einzugreifen und somit letztendlich den tumorzellenspezifisch veränderten Stoffwechsel zu stören und zu einem Absterben oder zumindest einer Wachstumshemmung der Tumorzellen, insbsondere aber einer Hemmung der Transformation zu Tumorzellen, beizutragen.

Im Rahmen der Erfindung kann es sich empfehlen, im Vorfeld einer Behandlung mit einer erfindungsgemäßen pharmazeutischen Zusammensetzung eine Probe aus einem Gewebe, welches als Tumorgewebe mit anderen Methoden identifiziert ist, zu entnehmen und die Gewebeprobe auf Expression bzw. Überexpression von Metaring zu untersuchen. Alternativ kann mit einer erfindungsgemäßen Detektorsubstanz zur Diagnose in vivo auf Metaring Abhängigkeit getestet werden. Wird eine Expression bzw. Überexpression von Metaring gegenüber Normalgewebe gleichen Typs festgestellt, so ist die Anwendung der erfindungsgemäßen pharmazeutischen Zusammensetzung indiziert.

Generell ist es im Rahmen der Erfindung möglich, patientenspezifisch auf differentielle Expression zu untersuchen, wobei Normalgewebeprobe und Tumorgewebeproben bzw. tumorverdächtige Gewebeproben dem (gleichen) Patienten entnommen und vergleichend auf Werte der Metaring Expression untersucht werden.

Handelt es sich bei dem Tumor um einem Typus, bei welchem Tumorzellen Metaring exprimieren, Normalzellen gleichen Gewebetyps jedoch nicht, so ist es besonders bevorzugt, wenn die an Metaring bindende Substanz zusätzlich eine zytotoxische und/oder immunstimulierende Komponente trägt. Dies führt dann letztendlich dazu, dass praktisch ausschließlich Tumorzellen getötet werden, sei es durch die Zytotoxizität, sei es durch Angriff durch das stimulierte Immunsystem, während Normalzellen in dem Gewebe praktisch vollständig erhalten bleiben. In dieser Ausführungsform braucht die bindende Substanz selbst nicht inhibierend auf Metaring zu wirken, da die bindende Substanz dann lediglich als Marker funktionieren muß, welcher die Komponenten zu Ziel-Tumorzellen trägt. Im Falle des Einsatzes einer zytotoxischen Komponente wird es sich besonders empfehlen, wenn die pharmazeutische Zusammensetzung zur lokalen Applikation in Tumorzellen enthaltendem Gewebe hergerichtet ist, beispielsweise zur Injektion.

Schließlich betrifft die Erfindung eine Metaring Nukleinsäure oder ein MetaRing Protein oder Peptid. Im Falle der Nukleinsäure (SEQ-ID1) ist von besonderem Interesse die Teilsequenz beginnend bei 169 bis zum Stop Codon TGA (SEQ-ID2). Das Protein (SEQ-ID3) weist eine RING Finger Domäne vom Typ C3H2C3 auf (SEQ-ID4). Darüber hinaus enthält die Sequenz (SEQ-ID3) ein Signalpeptid (AS 1-33), eine Glutamat-reiche Region (AS 772-780), zwei Prolin-reiche Regionen (AS 360-374 und 576-597) eine Serin-reiche Region (AS 464-503) sowie eine Histidin-reiche Region (AS 544-558).

Definitionen und weitere Ausführungsformen der Erfindung.

Metaring Sequenzen, insbesondere Teilsequenzen, welche in im Rahmen der Erfindung nutzbare Nukleinsäuren oder Peptide oder Proteine enthalten sind, sind in den Seq.-ID 1 bis 4 (siehe auch die Figuren) dargestellt. Die im Rahmen der Erfindung nutzbaren Nukleinsäuren oder Peptide oder Proteine können auch aus diesen Sequenzen bestehen.

Im Rahmen dieser Beschreibung wird die Bezeichnung Metaring für alle humanen Isoformen, bekannt oder neu, auf Nukleinsäuren- oder Aminosäurenbasis, verwendet. Mit diesen Begriffen mit umfaßt sind auch die im Rahmen dieser Beschreibung offenbarten kurzen Sequenzen, beispielsweise Immunisierungssequenzen. Weiterhin mit umfaßt sind auch Homologe, wobei die Homologie zumindest 80%, vorzugsweise mehr als 90%, höchstvorzugsweise mehr als 95%, beträgt, berechnet mit dem Programm MEGALIGN (DNASTAR LASERGENE) in der zum Zeitpunkt der vorliegenden Anmeldung aktuellen Fassung. Im Falle der Nukleinsäuresequenzen sind auch komplementäre oder allelische Varianten mit umfaßt. Weiterhin sind Sequenzen umfaßt, welche lediglich Teilsequenzen der explizit offenbarten Sequenzen, beispielsweise ein Exon oder mehrere Exons, oder komplementärer Sequenzen hierzu darstellen, mit der Maßgabe, daß diese Teilsequenzen im Falle der Nukleinsäuren eine für eine Hybridisierung mit einer erfindungsgemäßen Nukleinsäure hinreichende Länge, zumindest 50 Basen, aufweisen und im Falle der Proteine bzw. Peptide mit zumindest gleicher Affinität an ein protein- oder peptidspezifisches Zielmolekül binden. Weiterhin sind alle mit erfindungsgemäßen Nukleinsäuren hybridisierende Nukleinsäuren umfaßt, nämlich solche, die unter stringenten Bedingungen (5°C bis 25°C unterhalb der Aufschmelztemperatur; siehe ergänzend J.M. Sambrook et al., A laboratory manual, Cold Spring Harbor Laboratory Press (1989) und E.M. Southern, J Mol Biol, 98:503ff (1975)) hybridisieren. Es versteht sich, daß die Erfindung auch Expressionskassetten umfaßt, i.e. eine oder mehrere der erfindungsgemäßen Nukleinsäuresequenzen mit mindestens einer operativ verbundenen Kontroll- oder regulatorischen Sequenz. Eine solche Expressionskassette kann auch eine Sequenz für ein bekanntes Protein umfassen, wobei im Zuge der Translation ein Fusionsprotein aus einem bekannten Protein und einem erfindungsgemäßen Protein oder Peptid entsteht. Ebenso sind auch antisense Sequenzen zu den vorstehenden Nukleinsäuresequenzen umfaßt. Schließlich sind RNA sowie damit korrelierende DNA und umgekehrt umfaßt, ebenso wie genomische DNA als auch korrelierte cDNA und umgekehrt.

Im Zusammenhang mit erfindungsgemäßen Verwendungen umfassen die Begriffe der Metaring Nukleinsäuren oder Protein bzw. Peptide neben den Volllängen der offenbarten Sequenzen (siehe auch vorstehender Absatz) auch Teilsequenzen hieraus, und zwar mit einer Mindestlänge von 12 bis 30 Nukleotiden, vorzugsweise 30 bis 90 Nukleotiden, im Falle der Nukleinsäuren und einer Mindestlänge von 4 bis 10 Aminosäuren, vorzugsweise 10 bis 30 Aminosäuren, im Falle der Peptide oder Proteine. Diese Teilsequenzen können in ansonsten von Metaring verschiedene Nukleinsäuren- oder Protein- bzw. Peptidsequenzen eingebaut sein.

Der Begriff der Behandlung umfaßt auch die Prophylaxe, insbesondere die Prophylaxe der Progression zu Tumoren.

Als Inhibitor ist eine Verbindung oder Substanz bezeichnet, welche entweder die Bildung von Metaring Protein inhibiert oder gebildetes Metaring Protein in der Aktivität reduziert, bezogen auf die Metaring Aktivität in Abwesenheit des Inhibitors. Insofern kann ein Inhibitor einerseits eine Substanz sein, welche in der Entstehungskaskade von Metaring inhibierend eingreift. Auf der anderen Seite kann ein Inhibitor eine Substanz sein, welche mit gebildetem Metaring eine Bindung eingeht, und zwar dergestalt, dass weitere physiologische Wechselwirkungen mit endogenen Substanzen zumindest reduziert sind.

Mimikry-Moleküle sind Verbindungen, die den variablen Bereich, insbesondere den Bindungsbereich eines Antikörpers, nachbilden und an gleicher Stelle eines Zielmoleküls binden, wie der zu Grunde liegende Antikörper.

Der Begriff der Antikörper umfaßt polyklonale Antikörper, monoklonale Antikörper, nicht-humane, humane und humanisierte Antikörper, sowie Phage-Display-Antikörper, aber auch chimäre Antikörper sowie spezifische Fragmente der leichten und/oder der schweren Kette des variablen Bereiches zu Grunde liegender Antikörper vorstehender Art sowie anti-idiotypische Antikörper. Die Herstellung bzw. Gewinnung solcher Antikörper mit vorgegebenen Immunogenen ist dem Durchschnittsfachmann wohl vertraut und braucht nicht näher erläutert zu werden. Weiterhin umfaßt der Begriff der Antikörper bispezifische Antikörper. Bispezifische Antikörper kombinieren eine definierte Immunzellaktivität mit einer spezifischen Tumorzellerkennung, wodurch Tumorzellen getötet werden. Ein bispezifischer Antikörper bindet einerseits an ein Auslösemolekül der Immun-Effektorzelle (z.B. CD3, CD16, CD64) und andererseits an Antigene der Tumorzielzelle.

Die galenische Herrichtung einer erfindungsgemäßen pharmazeutischen Zusammensetzung kann in fachüblicher Weise erfolgen. Als Gegenionen für ionische Verbindungen kommen beispielsweise Na+, K+, Li+ oder Cyclohexylammonium infrage. Geeigente feste oder flüssige galenische Zubereitungsformen sind beispielsweise Granulate, Pulver, Dragees, Tabletten, (Mikro-) Kapseln, Suppositorien, Sirupe, Säfte, Suspensionen, Emulsionen, Tropfen oder injizierbare Lösungen (i.v., i.p., i.m.) sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als Hilfsstoffe sei Magnesiumcarbonat, Titandioxyd, Lactose, Mannit und andere Zucker, Talcum, Milcheiweiß, Gelatine, Stärke, Zellulose und ihre Derivate, tierische und pflanzliche Öle wie Lebertran, Sonnenblumen-, Erdnuss- oder Sesamöl, Polyethylenglycole und Lösungsmittel, wie etwa steriles Wasser und ein- oder mehrwertige Alkohole, beispielsweise Glycerin, genannt. Eine erfindungsgemäße pharmazeutische Zusammensetzung ist dadurch herstellbar, dass mindestens ein erfindungsgemäß verwendeter Inhibitor in definierter Dosis mit einem pharmazeutisch geeigneten und physiologisch verträglichen Träger und ggf. weiteren geeigneten Wirk-, Zusatz- oder Hilfsstoffen mit definierter Inhibitordosis gemischt und zu der gewünschten Darreichungsform hergerichtet ist.

Tumorzellen exprimieren Metaring differenziell, wenn Normalzellen des gleichen Gewebetyps (des gleichen oder verschiedener Probanden) dieses nicht exprimieren. Tumorzellen überexprimieren Metaring spezifisch bzw. differenziell, wenn Metaring im Vergleich zu Normalzellen des gleichen Gewebetyps zumindest in doppelter Menge exprimiert wird.

Zytotoxische Komponenten bzw. Gruppen sind Verbindungen, welche direkt oder indirekt Apoptose einleiten bzw. zu Nekrose führen oder zumindest wachstumshemmend wirken. Solche Gruppen bzw. Verbindungen können neben Radioisotopen (z.B. 188Re, 213Bi, 99mTc, 90Y, 131J, 177Lu) insbesondere Zytostatika sein, welche in der Tumortherapie eingesetzt werden. Beispiele hierfür sind: Alkylantien (z.B. Mechlorethamin, Ifosfamid, Chlorambucil, Cyclophosphamid, Melphalan, Alkylsulfonate, Busulphan, Nitrosoharnstoffe, Carmustin, Lomustin, Semustin, Triazene, Dacarbazin), Antimetaboliten (z.B. Folsäure-Antagonisten, Methotrexat, Pyrimidin-Analoga, Fluoruracil, Fluordesoxyuridin, Cytarabin, Gemcitabin, Purin-Analoga, Mercaptopurin), Mitosehemmer (z.B. Vincaalkaloide, Voncristin, Vinblastin, Paclitaxal, Docetaxel, Protaxel), Epipodophyllotoxine (z.B. Etoposid, Teniposid), Antibiotika (z.B. Dactinomycin, Daunorubicin, Idarubicin, Anthracycline, Bleomycin, L-Asparaginase), Platinkomplexverbindungen (z.B. Cisplatin), Hormone und verwandte Verbindungen (z.B. Nebennierenrindensteroide, Aminogluthetimid, Gestagene, Östrogene, Androgene, Antiöstrogene, Tamoxifen, Steriodanaloga, Flutamid). Bei Bindung einer solchen Verbindung mit einer an Metaring bindenden Substanz erfolgt die Kopplung dergestalt, daß die Affinität zu Metaring um nicht mehr als 90%, vorzugsweise 50%, bezogen auf die Substanz ohne zytostatische Gruppe, reduziert ist und die zytostatische Wirkung der Gruppe um nicht mehr als 90%, vorzugsweise 50%, bezogen auf die Verbindung ohne Substanz, reduziert ist.

Eine immunstimulierende Komponente ist meist ein Protein oder ein wirksamer Bestandteil hiervon, welches Zellen des Immunsystems stimuliert. Beispiele hierfür sind: Zytokine, wie M-CSF, GM-CSF, G-CSF, Interferone, wie IFN-alpha, -beta, -gamma, Interleukine wie IL-1 bis -16 (außer -8), human LIF, Chemokine wie Rantes, MCAF, MIP-1-alpha, -beta, NAP-1 und IL-8.

Eine Reportergruppe ist ein Atom, Molekül oder eine Verbindung, welche in Verbindung mit einem hierauf abgestellten Assay den Nachweis der Reportergruppe und der somit mit der Reportergruppe verbundenen Verbindung oder Substanz ermöglicht. Beispiele für Reportergruppen und hiermit assoziierte Detektionsmethoden sind: 32P-Labeling und Intensitätsmessung mittels Phosphoimager. Viele weitere Beispiele sind dem Durchschnittsfachmann bekannt und bedürfen nicht der detaillierten Aufzählung.

Eine an Metaring bindende Substanz kann eine Substanz sein, welche an ein Metaring Protein oder eine Metaring RNA bindet.

Im Rahmen der vorstehenden Definition gegenüber dem engen Wortsinn erweiterte Begriffsbestimmungen umfassen auch die bestimmten Begriffe im engen Wortsinn.

### Beispiele.

Im Folgenden wird die Erfindung anhand von lediglich bevorzugte Ausführungsformen darstellenden Beispielen und Figuren näher erläutert.
Es zeigen:

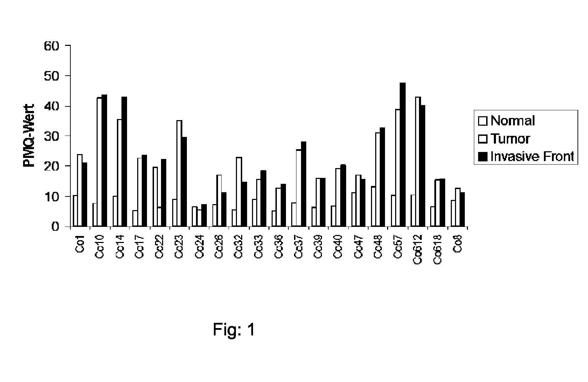

Fig. 1: Chipanalyse zur differentiellen Expression von Metaring in Colontumorgewebe aus 20 Patienten,

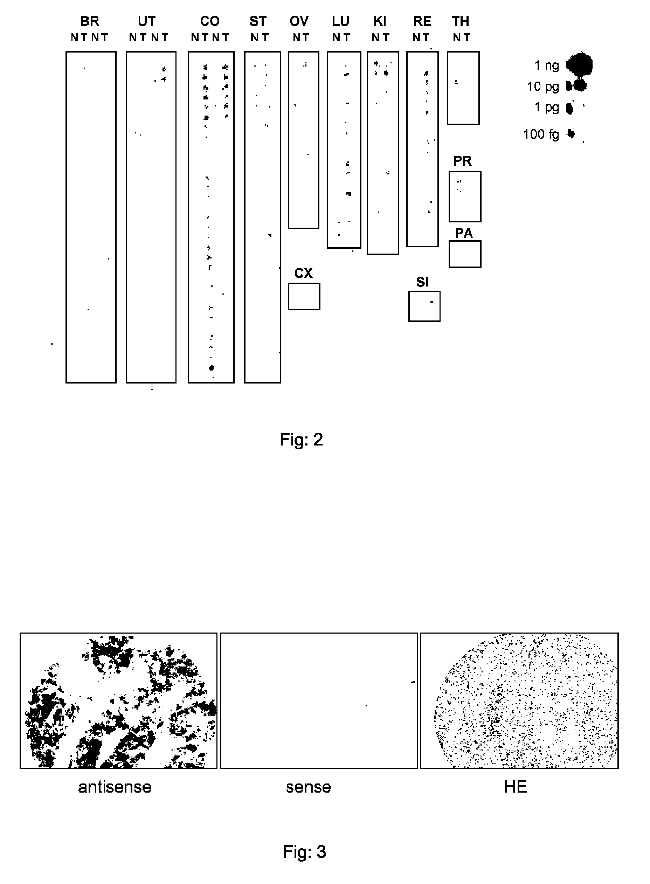

Fig. 2: Krebsgewebe Profiling (CPA)

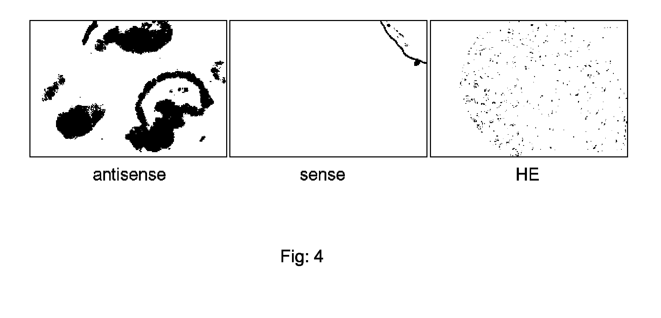

Fig. 3 und 4: in situ Hybridisierung von Kolon Tumorgeweben.

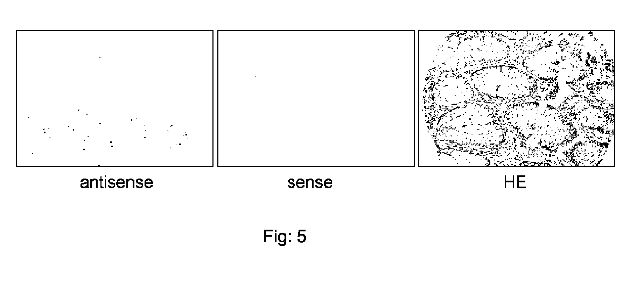

Fig. 5: in situ Hybridisierung von Kolon Normalgewebe.

### Beispiel 1: Untersuchte Gewebeproben

Es wurde Kolontumorgewebe von 26 Patienten mit zum Zeitpunkt der Erhebung invasiven Tumoren entnommen, und zwar einerseits aus dem Zentraltumor und andererseits aus der Invasionsfront. Zu Vergleichszwecken wurde zugleich Normalgewebe den Patienten entnommen. Die drei Gewebeprobentypen (Kerntumor, Invasionsfront, Normalgewebe) aus jeweils einem Patienten wurden einander zugeordnet. Im Einzelnen wurde wie folgt verfahren. Die Tumor- und -normalgewebeproben wurden gefroren und in 10µm Proben geschnitten. Aus jedem Patienten wurden zumindest 30 Proben gewonnen. Normale und maligne Bereiche wurden durch einen Pathologen mit Hilfe eines Mikroskopes identifiziert und markiert. Hierbei wird ggf. auch die Verlaufsform identifiziert und der Probe zugeordnet. Die jeweiligen Bereiche wurden unter dem Mikroskop resektiert unter Verwendung einer Nadel und jeweils separat auf -80°C eingefroren in 150µl GTC Puffer enthaltend 2% ß-Mercaptoethanol.

Für das Cancer-Profiling-Array wurden Tumor- und Normalgewebeproben aus Brust (BR), Uterus (UT), Kolon (CO), Dünndarm (Sl), Magen (ST), Ovar (OV), Lunge LU), Niere (Kl), Rektum (RE), Schilddrüse (TH), Prostata (PR), Pancreas (PA), und Zervix (CX) verwendet.

### Beispiel 2: Expressionsprofile der untersuchten Gewebe

Die Proben aus Beispiel 1 wurden einer Expressionsanalyse auf Metaring mittels der GeneChip-Technologie (Affimetrix) unterworfen. Dabei wird aus Proben aus Beispiel 1 RNA isoliert, amplifiziert und markiert. Die so erhaltene RNA wird einem Genchip aufgegeben, welcher eine Vielzahl von verschiedenen Oligonukleotiden enthält, wobei jeweils eines (oder auch mehrere, zu Kontrollzwecken) für ein definiertes Gen repräsentativ ist, i.e. eine charakteristische Teilsequenz hieraus aufweist. Man erhält sowohl qualitative, wie auch quantitative Information, ob eine betreffende Normalund/oder Tumorprobe ein betreffendes Gen exprimiert, und zwar auch im Verhältnis Tumor/Normal. Die Analyse ergab eine deutliche Überexpression von Metaring sowohl in der Invasionsfront des Tumors (76% bzw. 16/21) als auch im Kolon Adenokarzinom (69% bzw. 18/26) selbst. In Normalgewebe war die Metaring mRNA Expression in diesen Fällen mindestens um den Faktor 2 geringer. In der Fig. 1 sind exemplarisch Ergebnisse von gewebeproben von 20 Patienten (Patienten Nummern Co1. Co10 etc.) dargestellt. Von jedem Patienten wurde jeweils normales Kolongewebe (weiße Balken), Kolon Tumorgewebe (graue Balken) und invasive Front des Tumors (schwarze Balken) analysiert. In jeweils 15/20 Fällen ist in diesen proben die Metaring RNA Expression der Tumorproben bzw. der invasiven Front mindestens doppelt so hoch, wie in dem zugehörigen Normalgewebe desselben Patienten.

### Beispiel 3: Überexpression von Metaring in verschiedenen Tumorgeweben.

Ein Cancer Profiling Array (CPA, Clontech) mit 240 augetragenen humanen cDNAs von Tumor- und korrespondierenden Normalgeweben jeweils eines Patienten wurde unter Verwendung einr 32P-markierten Metaring-spezifischen Sonde unter Einsatz eines Phosphoimagers analysiert. Im Einzelnen wurden die proben aus Beispiel 1 untersucht.

Der Figur 2, welche einen Northern Blot anhand des Cancer-Profiling-Arrays darstellt, entnimmt man, dass eine differenzielle Expression in nahezu allen untersuchten Tumorgeweben (T=Tumor, N=Normal). Oben rechts ist eine Labelling Kontrolle für die Hybridisierung wiedergegeben; 1 pg Metaring plasmid DNA wurde mittels der gelabelten Probe problemlos detektiert. Tabelle 1 quantifiziert die Ergebnisse der Figur 2. Dabei wurde eine differenzielle Expression (pos) angenommen bei zumindest 2-facher Überexpression von Metaring in Tumorgewebe gegenüber Normalgewebe.

Tabelle 1

Tumor Überexpression abs. Überexpression rel.

Pos Anzahl/Gesamtanz. (%)

Brust 43/53 81 %

Kolon 37/39 95%

Lunge 17/21 81 %

Ovar 11/16 69%

Endometr. 39/44 89%

Magen 14/27 52%

Rektum 14/19 74%

### Beispiel 4: Immunhistochemischer Nachweis von Tumorzellen oder Nachweis mittels in situ Hybridisierung.

Gewebe wird aus einem Patienten mit Krebs oder dem Verdacht auf Krebs isoliert und als Paraffin- bzw. Gefrierschnitte präpariert. Diese Schnitte werden für den immunhistochemischen Nachweis mit einem gegen ein erfindungsgemäßes Protein gerichteten Antikörper auf die Überexpression des Proteins in Zellen untersucht. Es kann sich beispielsweise um polyklonale Antikörper handeln, die mittels einer der folgenden Peptide als Immunogen erhalten wurden.

SARHPQRKRRGGPS

VPLRRARPPDSSGSG

Ergebnisse einer in situ Hybridisierung sind in der Figur 3 dargestellt. Gewebeproben aus Colongewebe wurden mit einer Metaring spezifischen antisense Sonde inkubiert und mit BM-Purple gefärbt. Die Gegenfärbung erfolgte mit Kernecht-Rot. Es sind maligne und nichtmaligne Epithelzellen voneinander zu unterscheiden, von denen die malignen Zellen eine starke Anfärbung der RNA in der in situ Hybridisierung zeigen (Fig. 3 und 4). Man erkennt, dass Metaring im Colontumorgewebe (links) auf RNA Ebene deutlich überexprimiert ist, verglichen zur Kontrolle (sense, Mitte). Die rechten Bilder zeigen eine Hämatoxilin/Eosin-Färbung des entsprechenden Tumorareals. Fig. 5 zeigt eine entsprechende RNA in situ Hybridisierung von Kolon Normalgewebe. Nur 10% der Epithelzellen werden mit der antisense Sonde schwach gefärbt.

Die quantitative Auswertung der in situ Hybridisierung eines Arrays mit 53 Kolon Tumor Gewebeproben und 8 Kolon Normalgewebeproben ist in Tabelle 2 dargestellt. Dabei ist zu beachten, dass hier zwar viele Normalgewebe (7 von 8 Proben) eine positive Färbung aufweisen, diese jedoch in maximal 10% der Epithelzellen zu beobachten ist. In den positiv gefärbten Tumorgeweben sind dagegen immer 80-100% der Zellen gefärbt.

Tabelle 2

Färbung Tumor Probe Probe Normalgewebe

(Intens.)

negativ 10/53 (19%) 1/8 (13%)

+ 22/53 (41 %) 7*/8 (87%)

++ 19/53 (36%) 0/8 (0%)

+++ 2/53 (4%) 0/8 (0%)

### Beispiel 5: Erzeugung von anti-idiotypischen monoklonalen Antikörpern zu therapeutischen Zwecken

Ausgehend von einem erfindungsgemäß verwendeten Protein wird in fachüblicher Weise ein monoklonaler Antikörper Ab1 erzeugt, welcher in der Lage ist, das Protein spezifisch zu erkennen und daran zu binden. Dabei ist es unwesentlich, ob eine funktionale Domäne oder ein anderer zugänglicher Bereich erkannt wird. Mit Hilfe des erzeugten Antikörpers Ab1 wird in ebenso fachüblicher Weise ein zweiter anti-idiotypischer nicht humanisierter, beispielsweise Maus, monoklonaler Antikörper aAB1 erzeugt, welcher zur Herstellung einer pharmazeutischen Zusammensetzung zur Behandlung von Krebs, insbesondere der vorstehend genannten Tumoren, geeignet ist. Die Funktion des Antikörpers aAB1 beruht dabei darauf, dass dieser dem humanen Immunsystem ein Image des (humanen) Protein-Antigens gleichsam vortäuscht, wobei das Immunsystem den Antikörper aAB1 aufgrund seiner mangelnden Humanisierung als körperfremd erkennt. Der humane Körper bildet folglich eigene Antikörper, die gegen aAB1 und somit auch gegen das humane Protein bzw. dieses exprimierende Tumorzellen gerichtet sind.

### Beispiel 6: Metaring mRNA Knock-down durch RNAi oder antisense Oligos

Transfektion von Metaring spezifischen antisense-Oligonukleotiden oder siRNA-Molekülen in Zellen vermindert die intrazelluläre Menge an Metaring mRNA. Hierfür kommen beispielsweise die folgenden Oligos in Frage:

AATGCAGTCCCACCCACTGTA

AAAGGCCCATGTGAGGATTGA

AAGACTCCACCTCATTCGCCA

AAGCTATTGCACAGAACGCAG

AAGTCACCGGATCCAACTCAG

AAGAGGCTGCTACCAGAAACC

Tumorzellen werden mit einem dieser Oligos bzw. siRNA Moleküle transfiziert, nach 48 bis 72 Stunden werden die Zellen lysiert, RNA präpariert und die menge an metaring mRNA mittels Real-Time PCR Analyse quantifiziert. Die Transfektion eines Metaring spezifischen Ologinukleotides bewirkt eine Verringerung der Metaring RNA Menge, verglichen mit der Kontrolle, welche mit einem unspezifischen Oligo transfiziert wurde. Wirksame Oligos sind dann therapeutisch einsetzbar im Rahmen geeignet hergerichteter pharmazeutischer Zusammensetzungen.

SEQUENCE LISTING

<110>  Hinzmann, Bernd
       Staub, Eike
       Rosenthal, André
       Hermann, Klaus
       Heiden, Esmeralda
       Dahl, Edgar
       Klaman, Irina
       Mönch, Malve
       Stei, Martin

<120>  Verwendung von an Metaring bindenden Substanzen zur Diagnose und
       Behandlung von Krebs

<130>  MET/DE/0331

<140>  DE 103 45 011.4-41
<141>  2003-09-22

<160>  4

<170>  PatentIn version 3.2

<210>  1
<211>  3802
<212>  DNA
<213>  Homo sapiens

<400>  1
aaaaaaaaaa aactttagag aaaggaaggg ccaaaactac gacttggctt tctgaaacgg      60

aagcataaat gttcttttcc tccatttgtc tggatctgag aacctgcatt tggtattagc     120

tagtggaagc agtatgtatg gttgaagtgc attgctgcag ctggtagcat gagtggtggc     180

caccagctgc agctggctgc cctctggccc tggctgctga tggctaccct gcaggcaggc     240

tttggacgca caggactggt actggcagca gcggtggagt ctgaaagatc agcagaacag     300

aaagctgtta tcagagtgat ccccttgaaa atggacccca caggaaaact gaatctcact     360

ttggaaggtg tgtttgctgg tgttgctgaa ataactccag cagaaggaaa attaatgcag     420

tcccacccac tgtacctgtg caatgccagt gatgacgaca atctggagcc tggattcatc     480

agcatcgtca agctggagag tcctcgacgg gccccccgcc cctgcctgtc actggctagc     540

aaggctcgga tggcgggtga gcgaggagcc agtgctgtcc tctttgacat cactgaggat     600

cgagctgctg ctgagcagct gcagcagccg ctggggctga cctggccagt ggtgttgatc     660

tggggtaatg acgctgagaa gctgatggag tttgtgtaca agaaccaaaa ggcccatgtg     720

aggattgagc tgaaggagcc cccggcctgg ccagattatg atgtgtggat cctaatgaca     780

gtggtgggca ccatctttgt gatcatcctg gcttcggtgc tgcgcatccg gtgccgcccc     840

cgccacagca ggccggatcc gcttcagcag agaacagcct gggccatcag ccagctggcc     900

accaggaggt accaggccag ctgcaggcag gcccggggtg agtggccaga ctcagggagc     960

agctgcagct cagcccctgt gtgtgccatc tgtctggagg agttctctga ggggcaggag    1020

ctacgggtca tttcctgcct ccatgagttc catcgtaact gtgtggaccc ctggttacat 1080

cagcatcgga cttgccccct ctgcgtgttc aacatcacag agggagattc attttcccag 1140

tccctgggac cctctcgatc ttaccaagaa ccaggtcgaa gactccacct cattcgccag 1200

catcccggcc atgcccacta ccacctccct gctgcctacc tgttgggccc ttcccggagt 1260

gcagtggctc ggcccccacg acctggtccc ttcctgccat cccaggagcc aggcatgggc 1320

cctcggcatc accgcttccc cagagctgca catccccggg ctccaggaga gcagcagcgc 1380

ctggcaggag cccagcaccc ctatgcacaa ggctggggaa tgagccacct ccaatccacc 1440

tcacagcacc ctgctgcttg cccagtgccc ctacgccggg ccaggccccc tgacagcagt 1500

ggatctggag aaagctattg cacagaacgc agtgggtacc tggcagatgg gccagccagt 1560

gactccagct cagggccctg tcatggctct tccagtgact ctgtggtcaa ctgcacggac 1620

atcagcctac agggggtcca tggcagcagt tctactttct gcagctccct aagcagtgac 1680

tttgaccccc tagtgtactg cagccctaaa ggggatcccc agcgagtgga catgcagcct 1740

agtgtgacct ctcggcctcg ttccttggac tcggtggtgc ccacagggga aacccaggtt 1800

tccagccatg tccactacca ccgccaccgg caccaccact acaaaaagcg gttccagtgg 1860

catggcagga agcctggccc agaaaccgga gtcccccagt ccaggcctcc tattcctcgg 1920

acacagcccc agccagagcc accttctcct gatcagcaag tcaccggatc caactcagca 1980

gccccttcgg ggcggctctc taacccacag tgccccaggg ccctccctga gccagcccct 2040

ggcccagttg acgcctccag catctgcccc agtaccagca gtctgttcaa cttgcaaaaa 2100

tccagcctct ctgcccgaca cccacagagg aaaaggcggg ggggtccctc cgagcccacc 2160

cctggctctc ggccccagga tgcaactgtg cacccagctt gccagatttt tccccattac 2220

acccccagtg tggcatatcc ttggtcccca gaggcacacc ccttgatctg tggacctcca 2280

ggcctggaca agaggctgct accagaaacc ccaggcccct gttactcaaa ttcacagcca 2340

gtgtggttgt gcctgactcc tcgccagccc ctggaaccac atccacctgg ggaggggcct 2400

tctgaatgga gttctgacac cgcagagggc aggccatgcc cttatccgca ctgccaggtg 2460

ctgtcggccc agcctggctc agaggaggaa ctcgaggagc tgtgtgaaca ggctgtgtga 2520

gatgttcagg cctagctcca accaagagtg tgctccagat gtgtttgggc cctacctggc 2580

acagagtcct gctcctggga aaggaaagga ccacagcaaa caccattctt tttgccgtac 2640

ttcctagaag cactggaaga ggactggtga tggtggaggg tgagagggtg ccgtttcctg 2700

ctccagctcc agaccttgtc tgcagaaaac atctgcagtg cagcaaatcc atgtccagcc 2760

aggcaaccag ctgctgcctg tggcgtgtgt gggctggatc ccttgaaggc tgagtttttg 2820

agggcagaaa gctagctatg ggtagccagg tgttacaaag gtgctgctcc ttctccaacc 2880

cctacttggt ttccctcacc ccaagcctca tgttcatacc agccagtggg ttcagcagaa 2940

cgcatgacac cttatcacct ccctccttgg gtgagctctg aacaccagct ttggcccctc 3000

cacagtaagg ctgctacatc aggggcaacc ctggctctat cattttcctt ttttgccaaa 3060

aggaccagta gcataggtga gccctgagca ctaaaaggag gggtccctga agctttccca 3120

ctatagtgtg gagttctgtc cctgaggtgg gtacagcagc cttggttcct ctgggggttg 3180

agaataagaa tagtggggag ggaaaaactc ctccttgaag atttcctgtc tcagagtccc 3240

agagaggtag aaaggaggaa tttctgctgg actttatctg ggcagaggaa ggatggaatg 3300

aaggtagaaa aggcagaatt acagctgagc ggggacaaca aagagttctt ctctgggaaa 3360

agttttgtct tagagcaagg atggaaaatg gggacaacaa aggaaaagca aagtgtgacc 3420

cttgggtttg gacagcccag aggcccagct ccccagtata agccatacag gccagggacc 3480

cacaggagag tggattagag cacaagtctg gcctcactga gtggacaaga gctgatgggc 3540

ctcatcaggg tgacattcac cccagggcag cctgaccact cttggcccct caggcattat 3600

cccatttgga atgtgaatgt ggtggcaaag tgggcagagg accccacctg ggaacctttt 3660

tccctcagtt agtggggaga ctagcaccta ggtacccaca tgggtattta tatctgaacc 3720

agacagacgc ttgaatcagg cactatgtta agaaatatat ttatttgcta atatatttat 3780

ccacaaaaaa aaaaaaaaaa aa 3802

<210> 2
<211> 2352
<212> DNA
<213> Homo sapiens

<400> 2
atgagtggtg gccaccagct gcagctggct gccctctggc cctggctgct gatggctacc 60

ctgcaggcag gctttggacg cacaggactg gtactggcag cagcggtgga gtctgaaaga 120

tcagcagaac agaaagctgt tatcagagtg atccccttga aaatggaccc cacaggaaaa 180

ctgaatctca ctttggaagg tgtgtttgct ggtgttgctg aaataactcc agcagaagga 240

aaattaatgc agtcccaccc actgtacctg tgcaatgcca gtgatgacga caatctggag 300

cctggattca tcagcatcgt caagctggag agtcctcgac gggccccccg cccctgcctg 360

tcactggcta gcaaggctcg gatggcgggt gagcgaggag ccagtgctgt cctctttgac 420

atcactgagg atcgagctgc tgctgagcag ctgcagcagc cgctggggct gacctggcca 480

gtggtgttga tctggggtaa tgacgctgag aagctgatgg agtttgtgta caagaaccaa 540

aaggcccatg tgaggattga gctgaaggag cccccggcct ggccagatta tgatgtgtgg 600

atcctaatga cagtggtggg caccatcttt gtgatcatcc tggcttcggt gctgcgcatc 660

cggtgccgcc cccgccacag caggccggat ccgcttcagc agagaacagc ctgggccatc 720

agccagctgg ccaccaggag gtaccaggcc agctgcaggc aggcccgggg tgagtggcca 780

gactcaggga gcagctgcag ctcagcccct gtgtgtgcca tctgtctgga ggagttctct 840

gaggggcagg agctacgggt catttcctgc ctccatgagt tccatcgtaa ctgtgtggac 900

ccctggttac atcagcatcg gacttgcccc ctctgcgtgt tcaacatcac agagggagat 960

tcattttccc agtccctggg accctctcga tcttaccaag aaccaggtcg aagactccac 1020

ctcattcgcc agcatcccgg ccatgcccac taccacctcc ctgctgccta cctgttgggc 1080

ccttcccgga gtgcagtggc tcggccccca cgacctggtc ccttcctgcc atcccaggag 1140

ccaggcatgg gccctcggca tcaccgcttc cccagagctg cacatccccg ggctccagga 1200

gagcagcagc gcctggcagg agcccagcac ccctatgcac aaggctgggg aatgagccac 1260

ctccaatcca cctcacagca ccctgctgct tgcccagtgc ccctacgccg ggccaggccc 1320

cctgacagca gtggatctgg agaaagctat tgcacagaac gcagtgggta cctggcagat 1380

gggccagcca gtgactccag ctcagggccc tgtcatggct cttccagtga ctctgtggtc 1440

aactgcacgg acatcagcct acagggggtc catggcagca gttctacttt ctgcagctcc 1500

ctaagcagtg actttgaccc cctagtgtac tgcagcccta aaggggatcc ccagcgagtg 1560

gacatgcagc ctagtgtgac ctctcggcct cgttccttgg actcggtggt gcccacaggg 1620

gaaacccagg tttccagcca tgtccactac caccgccacc ggcaccacca ctacaaaaag 1680

cggttccagt ggcatggcag gaagcctggc ccagaaaccg gagtccccca gtccaggcct 1740

cctattcctc ggacacagcc ccagccagag ccaccttctc ctgatcagca agtcaccgga 1800

tccaactcag cagccccttc ggggcggctc tctaacccac agtgccccag ggccctccct 1860

gagccagccc ctggcccagt tgacgcctcc agcatctgcc ccagtaccag cagtctgttc 1920

aacttgcaaa aatccagcct ctctgcccga cacccacaga ggaaaaggcg ggggggtccc 1980

tccgagccca cccctggctc tcggccccag gatgcaactg tgcacccagc ttgccagatt 2040

tttccccatt acacccccag tgtggcatat ccttggtccc cagaggcaca ccccttgatc 2100

tgtggacctc caggcctgga caagaggctg ctaccagaaa ccccaggccc ctgttactca 2160

aattcacagc cagtgtggtt gtgcctgact cctcgccagc ccctggaacc acatccacct 2220

ggggaggggc cttctgaatg gagttctgac accgcagagg gcaggccatg cccttatccg 2280

cactgccagg tgctgtcggc ccagcctggc tcagaggagg aactcgagga gctgtgtgaa 2340

caggctgtgt ga 2352

<210> 3
<211> 798
<212> PRT
<213> Homo sapiens

<400> 3

Trp Lys Gln Tyr Val Trp Leu Lys Cys Ile Ala Ala Ala Gly Ser Met
1               5                   10                  15

Ser Gly Gly His Gln Leu Gln Leu Ala Ala Leu Trp Pro Trp Leu Leu
            20                  25                  30

Met Ala Thr Leu Gln Ala Gly Phe Gly Arg Thr Gly Leu Val Leu Ala
        35                  40                  45

Ala Ala Val Glu Ser Glu Arg Ser Ala Glu Gln Lys Ala Val Ile Arg
    50                  55                  60

Val Ile Pro Leu Lys Met Asp Pro Thr Gly Lys Leu Asn Leu Thr Leu
65                  70                  75                  80

Glu Gly Val Phe Ala Gly Val Ala Glu Ile Thr Pro Ala Glu Gly Lys
                85                  90                  95

Leu Met Gln Ser His Pro Leu Tyr Leu Cys Asn Ala Ser Asp Asp Asp
            100                 105                 110

Asn Leu Glu Pro Gly Phe Ile Ser Ile Val Lys Leu Glu Ser Pro Arg
        115                 120                 125

Arg Ala Pro Arg Pro Cys Leu Ser Leu Ala Ser Lys Ala Arg Met Ala
    130                 135                 140

Gly Glu Arg Gly Ala Ser Ala Val Leu Phe Asp Ile Thr Glu Asp Arg
145                 150                 155                 160

Ala Ala Ala Glu Gln Leu Gln Gln Pro Leu Gly Leu Thr Trp Pro Val
                165                 170                 175

Val Leu Ile Trp Gly Asn Asp Ala Glu Lys Leu Met Glu Phe Val Tyr
            180                 185                 190

Lys Asn Gln Lys Ala His Val Arg Ile Glu Leu Lys Glu Pro Pro Ala
        195                 200                 205

Trp Pro Asp Tyr Asp Val Trp Ile Leu Met Thr Val Val Gly Thr Ile
    210                 215                 220

Phe Val Ile Ile Leu Ala Ser Val Leu Arg Ile Arg Cys Arg Pro Arg
225                 230                 235                 240

His Ser Arg Pro Asp Pro Leu Gln Gln Arg Thr Ala Trp Ala Ile Ser
                245                 250                 255

Gln Leu Ala Thr Arg Arg Tyr Gln Ala Ser Cys Arg Gln Ala Arg Gly
            260                 265                 270

Glu Trp Pro Asp Ser Gly Ser Ser Cys Ser Ser Ala Pro Val Cys Ala
        275                 280                 285

Ile Cys Leu Glu Glu Phe Ser Glu Gly Gln Glu Leu Arg Val Ile Ser
    290                 295                 300

Cys Leu His Glu Phe His Arg Asn Cys Val Asp Pro Trp Leu His Gln
305                 310                 315                 320

His Arg Thr Cys Pro Leu Cys Val Phe Asn Ile Thr Glu Gly Asp Ser
                325                 330                 335

Phe Ser Gln Ser Leu Gly Pro Ser Arg Ser Tyr Gln Glu Pro Gly Arg
            340                 345                 350

Arg Leu His Leu Ile Arg Gln His Pro Gly His Ala His Tyr His Leu
        355                 360                 365

Pro Ala Ala Tyr Leu Leu Gly Pro Ser Arg Ser Ala Val Ala Arg Pro
    370                 375                 380

Pro Arg Pro Gly Pro Phe Leu Pro Ser Gln Glu Pro Gly Met Gly Pro
385                 390                 395                 400

Arg His His Arg Phe Pro Arg Ala Ala His Pro Arg Ala Pro Gly Glu
                405                 410                 415

Gln Gln Arg Leu Ala Gly Ala Gln His Pro Tyr Ala Gln Gly Trp Gly
            420                 425                 430

Met Ser His Leu Gln Ser Thr Ser Gln His Pro Ala Ala Cys Pro Val
        435                 440                 445

Pro Leu Arg Arg Ala Arg Pro Pro Asp Ser Ser Gly Ser Gly Glu Ser
    450                 455                 460

Tyr Cys Thr Glu Arg Ser Gly Tyr Leu Ala Asp Gly Pro Ala Ser Asp
465                 470                 475                 480

Ser Ser Ser Gly Pro Cys His Gly Ser Ser Ser Asp Ser Val Val Asn
                485                 490                 495

Cys Thr Asp Ile Ser Leu Gln Gly Val His Gly Ser Ser Ser Thr Phe

500                 505                 510

Cys Ser Ser Leu Ser Ser Asp Phe Asp Pro Leu Val Tyr Cys Ser Pro
        515                 520                 525

Lys Gly Asp Pro Gln Arg Val Asp Met Gln Pro Ser Val Thr Ser Arg
    530                 535                 540

Pro Arg Ser Leu Asp Ser Val Val Pro Thr Gly Glu Thr Gln Val Ser
545                 550                 555                 560

Ser His Val His Tyr His Arg His Arg His His His Tyr Lys Lys Arg
                565                 570                 575

Phe Gln Trp His Gly Arg Lys Pro Gly Pro Glu Thr Gly Val Pro Gln
            580                 585                 590

Ser Arg Pro Pro Ile Pro Arg Thr Gln Pro Gln Pro Glu Pro Pro Ser
        595                 600                 605

Pro Asp Gln Gln Val Thr Gly Ser Asn Ser Ala Ala Pro Ser Gly Arg
    610                 615                 620

Leu Ser Asn Pro Gln Cys Pro Arg Ala Leu Pro Glu Pro Ala Pro Gly
625                 630                 635                 640

Pro Val Asp Ala Ser Ser Ile Cys Pro Ser Thr Ser Ser Leu Phe Asn
                645                 650                 655

Leu Gln Lys Ser Ser Leu Ser Ala Arg His Pro Gln Arg Lys Arg Arg
            660                 665                 670

Gly Gly Pro Ser Glu Pro Thr Pro Gly Ser Arg Pro Gln Asp Ala Thr
        675                 680                 685

Val His Pro Ala Cys Gln Ile Phe Pro His Tyr Thr Pro Ser Val Ala
    690                 695                 700

Tyr Pro Trp Ser Pro Glu Ala His Pro Leu Ile Cys Gly Pro Pro Gly
705                 710                 715                 720

Leu Asp Lys Arg Leu Leu Pro Glu Thr Pro Gly Pro Cys Tyr Ser Asn
                725                 730                 735

Ser Gln Pro Val Trp Leu Cys Leu Thr Pro Arg Gln Pro Leu Glu Pro
            740                 745                 750

His Pro Pro Gly Glu Gly Pro Ser Glu Trp Ser Ser Asp Thr Ala Glu
        755                 760                 765

Gly Arg Pro Cys Pro Tyr Pro His Cys Gln Val Leu Ser Ala Gln Pro
    770                 775                 780

Gly Ser Glu Glu Glu Leu Glu Glu Leu Cys Glu Gln Ala Val
785                 790                 795

<210>  4
<211>  41
<212>  PRT
<213>  Homo sapiens

<220>
<221>  MISC_FEATURE
<222>  (1)..(41)
<223>  RING Finger domain

<400>  4

Cys Ala Ile Cys Leu Glu Glu Phe Ser Glu Gly Gln Glu Leu Arg Val
1               5                   10                  15

Ile Ser Cys Leu His Glu Phe His Arg Asn Cys Val Asp Pro Trp Leu
            20                  25                  30

His Gln His Arg Thr Cys Pro Leu Cys
        35                  40

Sequenzprotokoll - freier Text

>SEQ ID 1

AAAAAAAAAAAACTTTAGAGAAAGGAAGGGCCAAAACTACGACTTGGCT

TTCTGAAACGGAAGCATAAAT

GTTCTTTTCCTCCATTTGTCTGGATCTGAGAACCTGCATTTGGTATTAG

CTAGTGGAAGCAGTATGTATG

GTTGAAGTGCATTGCTGCAGCTGGTAGCATGAGTGGTGGCCACCAGCT

GCAGCTGGCTGCCCTCTGGCCC
TGGCTGCTGATGGCTACCCTGCAGGCAGGCTTTGGACGCACAGGACT
GGTACTGGCAGCAGCGGTGGAGT
CTGAAAGATCAGCAGAACAGAAAGCTGTTATCAGAGTGATCCCCTTGA
AAATGGACCCCACAGGAAAACT
GAATCTCACTTTGGAAGGTGTGTTTGCTGGTGTTGCTGAAATAACTCCA
GCAGAAGGAAAATTAATGCAG
TCCCACCCACTGTACCTGTGCAATGCCAGTGATGACGACAATCTGGAG
CCTGGATTCATCAGCATCGTCA
AGCTGGAGAGTCCTCGACGGGCCCCCGCCCCTGCCTGTCACTGGCT
AGCAAGGCTCGGATGGCGGGTGA
GCGAGGAGCCAGTGCTGTCCTCTTTGACATCACTGAGGATCGAGCTGC
TGCTGAGCAGCTGCAGCAGCCG
CTGGGGCTGACCTGGCCAGTGGTGTTGATCTGGGGTAATGACGCTGA
GAAGCTGATGGAGTTTGTGTACA
AGAACCAAAAGGCCCATGTGAGGATTGAGCTGAAGGAGCCCCCGGCC
TGGCCAGATTATGATGTGTGGAT
CCTAATGACAGTGGTGGGCACCATCTTTGTGATCATCCTGGCTTCGGT
GCTGCGCATCCGGTGCCGCCCC
CGCCACAGCAGGCCGGATCCGCTTCAGCAGAGAACAGCCTGGGCCAT

CAGCCAGCTGGCCACCAGGAGGT

ACCAGGCCAGCTGCAGGCAGGCCCGGGGTGAGTGGCCAGACTCAGG

GAGCAGCTGCAGCTCAGCCCCTGT

GTGTGCCATCTGTCTGGAGGAGTTCTCTGAGGGGCAGGAGCTACGGG

TCATTTCCTGCCTCCATGAGTTC

CATCGTAACTGTGTGGACCCCTGGTTACATCAGCATCGGACTTGCCCC

CTCTGCGTGTTCAACATCACAG

AGGGAGATTCATTTTCCCAGTCCCTGGGACCCTCTCGATCTTACCAAG

AACCAGGTCGAAGACTCCACCT

CATTCGCCAGCATCCCGGCCATGCCCACTACCACCTCCCTGCTGCCTA

CCTGTTGGGCCCTTCCCGGAGT

GCAGTGGCTCGGCCCCCACGACCTGGTCCCTTCCTGCCATCCCAGGA

GCCAGGCATGGGCCCTCGGCATC

ACCGCTTCCCCAGAGCTGCACATCCCCGGCTCCAGGAGAGCAGCAG

CGCCTGGCAGGAGCCCAGCACCC

CTATGCACAAGGCTGGGGAATGAGCCACCTCCAATCCACCTCACAGCA

CCCTGCTGCTTGCCCAGTGCCC

CTACGCCGGGCCAGGCCCCCTGACAGCAGTGGATCTGGAGAAAGCTA

TTGCACAGAACGCAGTGGGTACC

TGGCAGATGGGCCAGCCAGTGACTCCAGCTCAGGGCCCTGTCATGGC

TCTTCCAGTGACTCTGTGGTCAA

CTGCACGGACATCAGCCTACAGGGGGTCCATGGCAGCAGTTCTACTTT

CTGCAGCTCCCTAAGCAGTGAC

TTTGACCCCCTAGTGTACTGCAGCCCTAAAGGGGATCCCCAGCGAGTG

GACATGCAGCCTAGTGTGACCT

CTCGGCCTCGTTCCTTGGACTCGGTGGTGCCCACAGGGGAAACCCAG

GTTTCCAGCCATGTCCACTACCA

CCGCCACCGGCACCACCACTACAAAAAGCGGTTCCAGTGGCATGGCA

GGAAGCCTGGCCCAGAAACCGGA

GTCCCCCAGTCCAGGCCTCCTATTCCTCGGACACAGCCCCAGCCAGA

GCCACCTTCTCCTGATCAGCAAG

TCACCGGATCCAACTCAGCAGCCCCTTCGGGGCGGCTCTCTAACCCA

CAGTGCCCCAGGGCCCTCCCTGA

GCCAGCCCCTGGCCCAGTTGACGCCTCCAGCATCTGCCCCAGTACCA

GCAGTCTGTTCAACTTGCAAAAA

TCCAGCCTCTCTGCCCGACACCCACAGAGGAAAGGCGGGGGGGTCC

CTCCGAGCCCACCCCTGGCTCTC

GGCCCCAGGATGCAACTGTGCACCCAGCTTGCCAGATTTTTCCCCATT

ACACCCCCAGTGTGGCATATCC

TTGGTCCCCAGAGGCACACCCCTTGATCTGTGGACCTCCAGGCCTGG

ACAAGAGGCTGCTACCAGAAACC

CCAGGCCCCTGTTACTCAAATTCACAGCCAGTGTGGTTGTGCCTGACT

CCTCGCCAGCCCCTGGAACCAC

ATCCACCTGGGGAGGGGCCTTCTGAATGGAGTTCTGACACCGCAGAG

GGCAGGCCATGCCCTTATCCGCA

CTGCCAGGTGCTGTCGGCCCAGCCTGGCTCAGAGGAGGAACTCGAGG

AGCTGTGTGAACAGGCTGTGTGA

GATGTTCAGGCCTAGCTCCAACCAAGAGTGTGCTCCAGATGTGTTTGG

GCCCTACCTGGCACAGAGTCCT

GCTCCTGGGAAAGGAAAGGACCACAGCAAACACCATTCTTTTTGCCGT

ACTTCCTAGAAGCACTGGAAGA

GGACTGGTGATGGTGGAGGGTGAGAGGGTGCCGTTTCCTGCTCCAGC

TCCAGACCTTGTCTGCAGAAAAC

ATCTGCAGTGCAGCAAATCCATGTCCAGCCAGGCAACCAGCTGCTGCC

TGTGGCGTGTGTGGGCTGGATC

CCTTGAAGGCTGAGTTTTTGAGGGCAGAAAGCTAGCTATGGGTAGCCA

GGTGTTACAAAGGTGCTGCTCC

TTCTCCAACCCCTACTTGGTTTCCCTCACCCCAAGCCTCATGTTCATAC

CAGCCAGTGGGTTCAGCAGAA

CGCATGACACCTTATCACCTCCCTCCTTGGGTGAGCTCTGAACACCAG

CTTTGGCCCCTCCACAGTAAGG

CTGCTACATCAGGGGCAACCCTGGCTCTATCATTTTCCTTTTTGCCAA

AAGGACCAGTAGCATAGGTGA

GCCCTGAGCACTAAAAGGAGGGGTCCCTGAAGCTTTCCCACTATAGTG

TGGAGTTCTGTCCCTGAGGTGG

GTACAGCAGCCTTGGTTCCTCTGGGGGTTGAGAATAAGAATAGTGGGG

AGGGAAAAACTCCTCCTTGAAG

ATTTCCTGTCTCAGAGTCCCAGAGAGGTAGAAAGGAGGAATTTCTGCT

GGACTTTATCTGGGCAGAGGAA

GGATGGAATGAAGGTAGAAAAGGCAGAATTACAGCTGAGCGGGGACA

ACAAAGAGTTCTTCTCTGGGAAA

AGTTTTGTCTTAGAGCAAGGATGGAAAATGGGGACAACAAAGGAAAAG

CAAAGTGTGACCCTTGGGTTTG

GACAGCCCAGAGGCCCAGCTCCCCAGTATAAGCCATACAGGCCAGGG

ACCCACAGGAGAGTGGATTAGAG

CACAAGTCTGGCCTCACTGAGTGGACAAGAGCTGATGGGCCTCATCAG

GGTGACATTCACCCCAGGGCAG

CCTGACCACTCTTGGCCCCTCAGGCATTATCCCATTTGGAATGTGAAT

GTGGTGGCAAAGTGGGCAGAGG

ACCCCACCTGGGAACCTTTTTCCCTCAGTTAGTGGGGAGACTAGCACC

TAGGTACCCACATGGGTATTTA

TATCTGAACCAGACAGACGCTTGAATCAGGCACTATGTTAAGAAATATA

TTTATTTGCTAATATATTTAT

CCACAAAAAAAAAAAAAAAAAA

>SEQ ID 2

atgagtggtggccaccagctgcagctggctgccctctggccctggctgctgatggctaccctgcaggcag

gctttggacgcacaggactggtactggcagcagcggtg

gagtctgaaagatcagcagaacagaaagctgttatcagagtgatccccttgaaaatggaccccacagg

aaaactgaatctcactttggaaggtgtgtttgctggtgttgctg

aaataactccagcagaaggaaaattaatgcagtcccacccactgtacctgtgcaatgccagtgatgacg

acaatctggagcctggattcatcagcatcgtcaagctggag

agtcctcgacgggcccccgcccctgcctgtcactggctagcaaggctcggatggcgggtgagcgagg

agccagtgctgtcctctttgacatcactgaggatcgagct

gctgctgagcagctgcagcagccgctggggctgacctggccagtggtgttgatctggggtaatgacgct

gagaagctgatggagtttgtgtacaagaaccaaaaggcc

catgtgaggattgagctgaaggagcccccggcctggccagattatgatgtgtggatcctaatgacagtgg

tgggcaccatctttgtgatcatcctggcttcggtgctgcgc

atccggtgccgcccccgccacagcaggccggatccgcttcagcagagaacagcctgggccatcagcc

agctggccaccaggaggtaccaggccagctgcaggcag

gcccggggtgagtggccagactcagggagcagctgcagctcagcccctgtgtgtgccatctgtctggag
gagttctctgaggggcaggagctacgggtcatttcctgc
ctccatgagttccatcgtaactgtgtggacccctggttacatcagcatcggacttgcccccctctgcgtgttca
acatcacagagggagattcattttcccagtccctgggacc
ctctcgatcttaccaagaaccaggtcgaagactccacctcattcgccagcatcccggccatgcccactac
cacctccctgctgcctacctgttgggcccttcccggagtgc
agtggctcggcccccacgacctggtcccttcctgccatcccaggagccaggcatgggccctcggcatca
ccgcttccccagagctgcacatccccgggctccaggag
agcagcagcgcctggcaggagcccagcacccctatgcacaaggctggggaatgagccacctccaat
ccacctcacagcaccctgctgcttgcccagtgcccctacgc
cgggccaggccccctgacagcagtggatctggagaaagctattgcacagaacgcagtgggtacctgg
cagatgggccagccagtgactccagctcagggccctgtc
atggctcttccagtgactctgtggtcaactgcacggacatcagcctacagggggtccatggcagcagttct
actttctgcagctccctaagcagtgactttgacccccctagt
gtactgcagccctaaaggggatccccagcgagtggacatgcagcctagtgtgacctctcggcctcgttcc
ttggactcggtggtgcccacaggggaaacccaggtttcc
agccatgtccactaccaccgccaccggcaccaccactacaaaaagcggttccagtggcatggcagga
agcctggcccagaaaccggagtcccccagtccaggcctc
ctattcctcggacacagccccagccagagccaccttctcctgatcagcaagtcaccggatccaactcag
cagccccttcggggcggctctctaacccacagtgccccag

ggccctccctgagccagcccctggcccagttgacgcctccagcatctgccccagtaccagcagtctgttc
aacttgcaaaaatccagcctctctgcccgacacccacag
aggaaaaggcggggggtccctccgagcccacccctggctctcggccccaggatgcaactgtgcacc
cagcttgccagatttttccccattacacccccagtgtggcat
atccttggtccccagaggcacacccccttgatctgtggacctccaggcctggacaagaggctgctaccag
aaaccccaggcccctgttactcaaattcacagccagtgtgg
ttgtgcctgactcctcgccagcccctggaaccacatccacctggggaggggccttctgaatggagttctga
caccgcagagggcaggccatgcccttatccgcactgcc
aggtgctgtcggcccagcctggctcagaggaggaactcgaggagctgtgtgaacaggctgtgtga

>SEQ ID 3

WKQYVWLKCIAAAGSMSGG

HQLQLAALWPWLLMATLQAGFGRTGLVLAAAVESERSAEQKAVIRVIPLK

MDPTGKLNLT

LEGVFAGVAEITPAEGKLMQSHPLYLCNASDDDNLEPGFISIVKLESPRRA

PRPCLSLAS

KARMAGERGASAVLFDITEDRAAAEQLQQPLGLTWPVVLIWGNDAEKLME

FVYKNQKAHV

RIELKEPPAWPDYDVWILMTVVGTIFVIILASVLRIRCRPRHSRPDPLQQRT

AWAISQLA

TRRYQASCRQARGEWPDSGSSCSSAPVCAICLEEFSEGQELRVISCLHEF

HRNCVDPWLH

QHRTCPLCVFNITEGDSFSQSLGPSRSYQEPGRRLHLIRQHPGHAHYHLP

AAYLLGPSRS

AVARPPRPGPFLPSQEPGMGPRHHRFPRAAHPRAPGEQQRLAGAQHPY

AQGWGMSHLQST

SQHPAACPVPLRRARPPDSSGSGESYCTERSGYLADGPASDSSSGPCH

GSSSDSVVNCTD

ISLQGVHGSSSTFCSSLSSDFDPLVYCSPKGDPQRVDMQPSVTSRPRSLD

SVVPTGETQV

SSHVHYHRHRHHHYKKRFQWHGRKPGPETGVPQSRPPIPRTQPQPEPP

SPDQQVTGSNSA

APSGRLSNPQCPRALPEPAPGPVDASSICPSTSSLFNLQKSSLSARHPQR

KRRGGPSEPT

PGSRPQDATVHPACQIFPHYTPSVAYPWSPEAHPLICGPPGLDKRLLPET

PGPCYSNSQP

VWLCLTPRQPLEPHPPGEGPSEWSSDTAEGRPCPYPHCQVLSAQPGSE

EELEELCEQAV

>SEQ ID 4

CAICLEEFSEGQELRVISCLHEFHRNCVDPWLHQHRTCPLC

## Patentansprüche

1. Verwendung einer für ein Metaring Peptid oder Protein codierenden Nukleinsäure und/oder eines Metaring Peptids oder Proteins zur Detektion von Krebs oder zur Detektion eines Risikos der Erkrankung an einem Tumor, wobei eine Gewebeprobe auf Transkription oder Übertranskription von Metaring RNA oder auf Expression oder Überexpression eines Metaring Proteins untersucht wird.

2. Verwendung nach Anspruch 1, wobei eine an für Metaring codierende Nukleinsäure oder eine an Metaring Protein oder Peptid bindende Detektorsubstanz, vorzugsweise enthaltend eine Reportergruppe, verwendet wird, wobei Bindung besagter Nukleinsäure und/oder besagten Proteins oder Peptids an die Detektorsubstanz halbquantitativ oder quantitativ detektiert wird.

3. Verwendung einer Metaring RNA oder eines Metaring Proteins oder Peptids zum Screenen nach daran bindenden Substanzen, insbesondere nach prospektiven Wirkstoffen zur Inhibierung von besagter RNA oder besagtem Protein oder Peptid oder nach prospektiven Detektorsubstanzen, wobei eine prospektive Substanz oder eine Mischung solcher prospektiver Substanzen mit besagter RNA oder besagtem Protein oder Peptid kontaktiert wird, wobei mit einem Bindungsassay Bindungsereignisse festgestellt werden, und wobei eine bindende prospektive Substanz, ggf. nach Dekonvolutierung, selektiert wird.

4. Verwendung einer Metaring inhibierenden oder daran bindenden Substanz zur Herstellung einer pharmazeutischen Zusammensetzung zur Behandlung von Krebs oder zur Herstellung einer pharmazeutischen Zusammensetzung zur Diagnose eines Tumors oder zur Diagnose eines Progressionsrisikos eines Tumors.

5. Verwendung nach Anspruch 4, wobei die Substanz ein Antikörper ist, welcher beispielsweise durch Immunisierung eines nicht-menschlichen Säugetiers mit einem Metaring Peptid oder Protein, mit Metaring transfizierten Zellen, oder einer hierfür für codierenden cDNA, erhältlich ist, oder ein Phage-Display Antikörper ist.

6. Verwendung nach Anspruch 4, wobei die Substanz eine Mimikriverbindung eines Antikörpers gegen ein Metaring Peptid oder Protein ist.

7. Verwendung nach Anspruch 4, wobei die Substanz, ein Aptamer, eine antisense RNA, eine siRNA, oder ein Ribozym ist.

8. Verwendung nach einem der Ansprüche 4 bis 7, wobei die Substanz zusätzlich eine zytotoxische und/oder immunstimulierende Komponente trägt.

9. Verwendung nach einem der Ansprüche 4 bis 8, wobei die pharmazeutische Zusammensetzung zur lokalen Applikation in Tumorzellen enthaltendem Gewebe hergerichtet ist.

10. Verfahren zur Diagnose einer Krebserkrankung, oder des Risikos der Erkrankung an einem solchen Tumor, wobei eine an Metaring bindende Detektorsubstanz in einer Ausführungsform mit einer Reportergruppe in zu untersuchendes Gewebe appliziert wird, wobei das zu untersuchende Gewebe dann einer Detektionsverfahrenstufe unterworfen wird, welche sensitiv für die Reportergruppe ist, und wobei im Fall der Detektion eines definierten Mindestwertes der Reportergruppe im Gewebe das Gewebe als Tumorzellen enthaltend oder als erkrankungsgefährdet qualifiziert wird.

11. Verfahren zur Behandlung von Krebs, wobei eine pharmazeutische Zusammensetzung nach einem der Ansprüche 4 bis 9 in einer physiologisch wirksamen Dosis und galenisch für die anzuwendende Darreichungsform hergerichtet einem Patienten dargereicht wird.

12. Protein oder Peptid enthaltend oder bestehend aus einer Sequenz Seq.-ID 3 oder 4, oder einer Teilsequenz der Mindestlänge von 4, 5, 6, 7, 8, 9, oder 10 AS aus besagten Sequenzen, oder Nukleinsäure codierend für ein vorstehend definiertes Protein oder Peptid, insbesondere enthaltend eine Teilsequenz von 12, 15, 18, 21, 24, 27, oder 30 Nukleotiden aus einer der Sequenzen SEQ-ID 1 oder 2.
